# EUROPÄISCHE PATENTSCHRIFT

(11) **EP 1 251 897 B1**
(45) Veröffentlichungstag und Bekanntmachung des Hinweises auf die Patenterteilung: **12.07.2006**
(21) Anmeldenummer: 00993166.8
(22) Anmeldetag: 20.11.2000
(51) Int. Cl.: A61M 21/00

(54) **VORRICHTUNG ZUM WECKEN EINER SCHLAFENDEN PERSON**
DEVICE FOR WAKING A SLEEPING PERSON
DISPOSITIF POUR REVEILLER UNE PERSONNE ENDORMIE

(30) Priorität: 20.11.1999 DE 19955876
(43) Veröffentlichungstag der Anmeldung: 30.10.2002
(73) Patentinhaber: Urbscheit, Hans-Jürgen, 40237 Düsseldorf (DE)
(72) Erfinder: Urbscheit, Hans-Jürgen, 40237 Düsseldorf (DE)
(86) Internationale Anmeldenummer: PCT/EP2000/011514
(87) Internationale Veröffentlichungsnummer: WO 2001/037913

(56) Entgegenhaltungen:
- DE-A- 19 926 873
- FR-A- 1 534 545
- FR-A- 2 710 010
- US-A- 4 725 824

## Beschreibung

Die Erfindung betrifft eine Vorrichtung zum Wecken einer schlafenden Person, bei der die Schlafdauer einstellbar ist, an deren Ende das Wecksignal startet.

Eine derartige Vorrichtung ist ein handelsüblicher Wecker, der zu einem bestimmten Zeitpunkt ein Wecksignal abgibt.
Aus der DE 198 11 206 A1 ist ein Wecker bekannt, der den Schlafzustand einer Person misst und in einem voreingestellten Weckzeitintervall die Person weckt, wenn die sog. REM-Schlafphase endet.
In der DE 195 09 478 A1 wird ein Weckverfahren offenbart, wobei der Weckvorgang nach Erreichen einer vorgegebenen Weckzeit innerhalb einer sog. REM-Schlafphase stattfindet.
Aus der DE 298 13 798 U1 ist eine Schlaf- und Weckkissen bekannt, welches nach einer voreingestellten Weckzeit eine schlafende Person aufweckt.

Die FR-A-1 534 545 beschreibt eine Wachhalte vorrichtung ohne einstellbare Schlafdauer.

Nachteilig an dieser Art der Weckvorrichtungen bzw. -verfahren ist, dass immer eine vordefinierte Weckzeit oder voreingestellte Schlafdauer vor Antritt des Schlafes an der Weckvorrichtung eingegeben werden muss. Bei keiner der Vorrichtungen beginnt die Schlafdauer mit genauem Eintritt des Schlafes.

So ist bekannt, dass der sog. Büroschlaf bzw. der sog. Mittagsschläfchen einer der gesündesten Schläfe ist. Trotz einer Vielzahl von Vorurteilen wurde von wissenschaftlicher Seite bestätigt, dass ein "Nickerchen" um die Mittagszeit aufbauend wirkt bzw. sehr gesund ist. Entscheidend ist jedoch der Zeitraum, die Zeitdauer von Schlafantritt bis Schlafende, d.h. zwischen dem genauen Einschlafzeitpunkt und dem Erwachen. Nachteilig ist bei allen bekannten Weckvorrichtungen, dass die Weckzeit vor dem Einschlafzeitpunkt eingestellt werden muss. Dies führt nicht nur zu Einschlafproblemen, sondern drängt den Erholungsschlaf in das ungesunde Abseits. D.h. der Schlafdauer wird nicht von dem Schlafzustand der Person bestimmt, sondern ist einzig davon abhängig, wann die Person einschläft.
Der Gedanke, dass man zum einen sofort einschlafen muss, um die Pause, in der man den Schlaf halten möchte, nicht zu überschreiten, und, dass man den Weckzeitpunkt kennt, bevor man eingeschlafen ist, führt zu einer eher stressigen Ruhephase, aber nicht zu einem erholsamen Schlaf, mit vordefinierter Schlafdauer.

Aufgabe der Erfindung ist es, eine konstruktiv einfache, preiswert herzustellende und einfach zu bedienende Weckvorrichtung der eingangs genannten Art zu schaffen, die den genauen Einschlafzeitpunkt einer Person misst und vom diesem Einschlafzeitpunkt an eine vordefinierte Schlafdauer in Gang setzt und nach deren Ablauf ein Wecksignal bzw. Weckimpuls auslöst.

Diese Aufgabe wird erfindungsgemäß dadurch gelöst, dass ein Messgerät vorgesehen ist, das den Einschlafzeitpunkt einer Person misst und dass die einstellbare Schlafdauer ab dem Einschlafzeitpunkt beginnt.

Eine derartige Vorrichtung stellt eine konstruktiv einfache, preiswert herzustellende und sehr einfach zu bedienende Vorrichtung zum Wecken dar, wobei eine genaue vordefinierte Schlafdauer exakt eingehalten wird. Durch das Messen des Einschlafzeitpunktes und die von da an laufende voreingestellte Schlafdauer wird garantiert, dass die Schlafphase zur tatsächlichen Erholung wird.

Eine derartige Vorrichtung ist insbesondere für kurze Schlafphasen, wie bei einem sog. Mittagsschläfchen, geeignet. Der Gedanke bzw. die Angst sofort einschlafen zu müssen, da man die Weckzeit bereits vorher eingestellt hat, und damit die innere Unruhe im Körper, kann durch eine erfindungsgemäße Vorrichtung ausgeschaltet werden.

Vorteilhaft ist es, wenn das Messgerät eine Empfangsvorrichtung zur Aufnahme von körpereigenen Signalen oder Reaktionen aufweist. Besonders günstig ist es, wenn die Empfangsvorrichtung zumindest einen Sensor oder Messfühler aufweist, der an oder in der Nähe des Körpers angeordnet ist.
Hierdurch können körpereigene Signale, Bewegungen oder Reaktionen registriert werden. Die Sensoren können direkt auf der Haut angeordnet sein.
So können körpereigene Signale des Benutzers der Vorrichtung zum Wecken gemessen werden, die den Beginn der Schlafphase anzeigen. Das Messgerät misst z.B. den Pulsschlag, den Pulsrhythmus, die Körpertemperatur, die Hautfeuchtigkeit, die Atemfrequenz, Atemvolumen oder Körpervibrationen. Diese von der Empfangsvorrichtung aufgezeichneten Messdaten werden ausgewertet, wodurch ein Einschlafzeitpunkt festgestellt werden kann.
Die Hautfeuchtigkeit kann beispielsweise durch die Hautleitfähigkeit, insbesondere durch elektrische Ströme bzw. Widerstände, bestimmt werden.
Spezielle Sensoren an dem Messgerät messen die gewünschten körpereigenen Signale.

Weiterhin günstig ist es, wenn das Messgerät, nach Registrierung eines bestimmten Grenzwertes, ein Signal abgibt, welches von einer Weckvorrichtung empfangen wird. Das Messgerät ist so programmiert, dass genau an dem Einschlafzeitpunkt, der Grenzwert erreicht wird und damit ein Signal an die Weckvorrichtung abgegeben wird. D.h. das Messgerät bestimmt selbständig den Zeitpunkt, an dem die Schlafphase beginnt.

Das Signal, welches von dem Messgerät abgegeben wird und durch die Weckvorrichtung empfangen wird, löst in der Weckvorrichtung den Beginn der voreingestellten Schlafdauer aus. Hierdurch wird gewährleistet, dass der Schlaf genau die Länge der vordefinierten Zeitdauer entspricht.
Nach Ablauf einer vorgegebenen Zeitspanne, z.B. 8 Minuten, 9 Sekunden, wird von der Weckvorrichtung der Weckvorgang eingeleitet. Die Zeitdauer der Schlafes ist beliebig variabel einprogrammierbar.
Vorteilhafterweise löst die Weckvorrichtung nach Ablauf der einstellbaren Schlafdauer ein Wecksignal bzw. Weckimpuls aus. Dabei kann das Wecksignal bzw. der Weckimpuls durch akustische, optische, mechanische oder elektrische Reize erfolgen. Die Weckvorrichtung kann z.B. Töne, Melodien, Vibrationen, Lichtspiele oder leichte Stromstöße am Ende der voreingestellten Schlafdauer auslösen. Dabei kann das Wecksignal bzw. der Weckimpuls von der Weckvorrichtung selbst oder von einem separaten Empfänger, Sensor oder Impulsgeber ausgehen. Der separate Empfänger, Sensor oder Impulsgeber ist idealerweise das gleiche Messgerät bzw. der gleiche Sensor, der schon die körpereigenen Signale gemessen hat.

Weiterhin von Vorteil ist es, wenn Messgerät und Weckvorrichtung in einem Bauteil angeordnet sind. Denkbar ist aber auch eine Trennung von Messgerät und Weckvorrichtung.

Günstig ist es wenn, das Messgerät und/oder die Weckvorrichtung in einer Uhr, einem Pulsmesser, einem Blutdruckmessgerät, einer Manschette, einer Brille, einem Kissen oder einem Schmuckelement angeordnet ist.
Bei der Manschette kann es sich wahlweise um eine Bein-, Arm-, Hals- oder Körpermanschette handeln. Ebenso eignet sich ein Klipp, insbesondere eine Ohrenklipp, als Träger für den Sensor des Messgerätes.
Das Messgerät kann ebenfalls ein Computer sein, der über eine entsprechende Empfangsvorrichtung und Software verfügt. Die Weckvorrichtung kann über die Software des Computers angesteuert werden und das entsprechende Signal auslösen. An einer Brille können Messfühler angeordnet sein. Ebenso kann eine Brille als optischer Wecksignalgeber dienen.

Vorteilhaft ist ebenso die Abgabe eines Duftstoffs nach Ablauf der einstellbaren Schlafdauer durch die Weckvorrichtung.

Die erfindungsgemäße Vorrichtung zum Wecken kann auch als Einschlafwarngerät verwendet werden, indem es z.B. Autofahrer, LKW-Fahrer, Wachpersonal etc. rechtzeitig auf Übermüdung hinweist bzw. bei einsetzendem Schlaf warnt oder weckt.

Die Schlafdauer kann auf wenige Sekunden oder Bruchteile von Sekunden eingestellt werden, dass sofort nach Eintritt des Übermüdung bzw. des Schlafes ein Wecksignal bzw. Weckimpuls ausgelöst wird.

## Patentansprüche

1. Vorrichtung zum Wecken einer schlafenden Person, bei der die Schlafdauer einstellbar ist, an deren Ende das Wecksignal ausgelöst wird, **dadurch gekennzeichnet, dass** ein Messgerät vorgesehen ist, das den Einschlafzeitpunkt einer Person misst, und dass die einstellbare Schlafdauer ab dem Einschlafzeitpunkt beginnt.

2. Vorrichtung nach Anspruch 1, **dadurch gekennzeichnet, dass** das Messgerät eine Empfangsvorrichtung zur Aufnahme von körpereigenen Signalen oder Reaktionen aufweist.

3. Vorrichtung nach Anspruch 1 und 2, **dadurch gekennzeichnet, dass** die Empfangsvorrichtung zumindest einen Sensor oder Messfühler aufweist, der an oder in der Nähe des Körpers angeordnet ist.

4. Vorrichtung nach einem der vorherigen Ansprüche, **dadurch gekennzeichnet, dass** das Messgerät, nach Registrierung eines bestimmten Grenzwertes, ein Signal abgibt, welches von einer Weckvorrichtung empfangen wird.

5. Vorrichtung nach einem der vorherigen Ansprüche, **dadurch gekennzeichnet, dass** in der Weckvorrichtung nach Empfang des Signals die einstellbare Schlafdauer zu laufen beginnt.

6. Vorrichtung nach einem der vorherigen Ansprüche, **dadurch gekennzeichnet, dass** die Weckvorrichtung nach Ablauf der einstellbaren Schlafdauer ein Wecksignal bzw. Weckimpuls abgibt.

7. Vorrichtung nach einem der vorherigen Ansprüche, **dadurch gekennzeichnet, dass** Messgerät und Weckvorrichtung in einem Bauteil angeordnet sind.

8. Vorrichtung nach einem der Ansprüche 1 bis 6, **dadurch gekennzeichnet, dass** Messgerät und Weckvorrichtung zwei getrennte Bauteile sind.

9. Vorrichtung nach einem der vorherigen Ansprüche, **dadurch gekennzeichnet, dass** das Messgerät und/oder die Weckvorrichtung in einer Uhr, einem Pulsmesser, einem Blutdruckmessgerät, einer Manschette, einer Brille, einem Kissen oder einem Schmuckelement angeordnet ist.

10. Vorrichtung nach einem der vorherigen Ansprüche, **dadurch gekennzeichnet, dass** die Weckvorrichtung nach Ablauf der einstellbaren Schlafdauer einen Duftstoff abgibt.

## Claims

1. A device for waking a sleeping person whereby the sleep time at the end of which a wake signal is emitted can be adjusted, **characterized in that** a monitoring unit is provided that determines when the person falls asleep and that triggers the start of the settable sleep time.

2. The device according to claim 1, **characterized in that** the monitoring unit has a receiver for the input of signals or reactions from the sleeper's body.

3. The device according to claim 1 or 2, **characterized in that** the receiver has at least one sensor or detector that is on or near the sleeper's body.

4. The device according to one of the preceding claims, **characterized in that** the monitoring unit, after recording a predetermined threshold value, emits a signal when is received by a waking unit.

5. The device according to one of the preceding claims, **characterized in that** the preset sleep time starts to run when the waking unit receives the signal.

6. The device according to one of the preceding claims, **characterized in that** the waking unit emits a waking signal or alarm after the preset sleep time runs out.

7. The device according to one of the preceding claims, **characterized in that** the monitoring unit and waking unit are in one assembly.

8. The device according to one of claim 1 to 6, **characterized in that** the monitoring unit is separate from the waking unit.

9. The device according to one of the preceding claims, **characterized in that** the monitoring unit and/or the waking unit are mounted in a watch, pulse detector, sphygmomanometer, a sleeve, a wrist glasses frame, a pillow, or a piece of jewelry.

10. The device according to one of the preceding claims, **characterized in that** the waking unit emits a vapor or mist after the preset times runs out.

## Revendications

1. Appareillage visant à réveiller une personne à la fin d'une période de sommeil pré réglée à l'aide d'un signal de réveil, **caractérisé en ce qu'**un instrument de mesure détecte l'instant d'entrée en sommeil et **en ce que** la durée du sommeil pré réglée commence à partir du moment d'entrée en sommeil.

2. Appareillage suivant la revendication no.1, **caractérisé en ce que** l'instrument de mesure contient un détecteur de signaux corporels ou de réactions biologiques.

3. Appareillage suivant la revendication no.1 et no.2, **caractérisé en ce que** l'instrument de détection de signaux dispose d'au moins une sonde ou d'un capteur se trouvant à même le corps ou à sa proximité.

4. Appareillage suivant l'une quelconque des revendications précédentes, **caractérisé en ce que** l'instrument de détection de signaux émet d'un signal après qu'une certaine valeur limite a été atteinte, lequel est reçu par un appareillage de réveil.

5. Appareillage suivant l'une quelconque des revendications précédentes, **caractérisé en ce que** l'appareillage de réveil déclenche la durée de sommeil préréglée après la réception du signal.

6. Appareillage suivant l'une quelconque des revendications précédentes, **caractérisé en ce qu'**à la fin de la durée de sommeil préréglée, l'appareillage de réveil émet un signal de réveil et/ou une impulsion de réveil.

7. Appareillage suivant l'une quelconque des revendications précédentes, **caractérisé en ce que** l'instrument de mesure et/ou l'appareillage de réveil sont intégrés dans un réceptacle.

8. Appareillage suivant l'une quelconque des revendications précédentes, **caractérisé en ce que** l'instrument de détection et l'appareillage de réveil représente deux éléments séparés.

9. Appareillage suivant l'une quelconque des revendications précédentes, **caractérisé en ce que** l'instrument de mesure et/ou l'appareillage de réveil est intégré dans une montre, un pulsomètre, un tensiomètre, une manchette, des lunettes, un coussin ou dans un élément de bijoux.

10. Appareillage suivant l'une quelconque des revendications précédentes, **caractérisé en ce que** l'appareillage de réveil dégage une matière odorante à la fin de la durée du sommeil préréglée.
